# EUROPEAN PATENT SPECIFICATION

(11) **EP 4 543 304 B1**
(45) Date of publication and mention of the grant of the patent: **11.11.2026**
(21) Application number: 23738188.4
(22) Date of filing: 18.06.2023
(51) Int. Cl.: A61B 10/00, A61B 5/15

(54) **SAMPLE QUANTITY VERIFICATION**
PROBENMENGENÜBERPRÜFUNG
VÉRIFICATION DE QUANTITÉ D'ÉCHANTILLONS

(30) Priority: 24.06.2022 US 202263355304 P; 28.10.2022 US 202263420272 P
(43) Date of publication of application: 30.04.2025
(73) Proprietor: Salignostics Ltd., 9112100 Jerusalem (IL)
(72) Inventor: DEUTSCH, Omer, 9062700 Ofra (IL); COHEN, Raluca, 9262227 Jerusalem (IL); NEUMANN, Yoav, 9987500 Tzur Hadassah (IL); KRIEF, Guy, 9654415 Jerusalem (IL)
(74) Representative: Potter Clarkson
(86) International application number: PCT/IL2023/050631
(87) International publication number: WO 2023/248215

(56) References cited:
- EP-A2- 0 520 408
- US-A1- 2003 064 526
- US-B2- 8 025 851

## Description

### CROSS-REFERENCE TO RELATED APPLICATIONS

The present application claims the priority of:
US Provisional application 63/355,304 to Deutsch et al., entitled "SAMPLE QUANTITY VERIFICATION," filed on June 24, 2022; and
US Provisional application 63/420,272 to Deutsch et al., entitled "SAMPLE QUANTITY VERIFICATION," filed on October 28, 2022.

Each of the above applications is assigned to the assignee of the present application.

### FIELD OF THE INVENTION

Some applications of the present invention relate in general to moisture detection. More specifically, some applications of the present invention relate to detecting a quantity of a body fluid.

### BACKGROUND

Diagnostic assays are performed on samples of body fluids taken from a subject, in order to detect the presence and/or concentration of various analytes in the sample, and to thereby determine a condition of the subject. It is important that such assays be sufficiently reliable.

International application PCT/IL2021/050697, published as WO 2021/250672, discloses an apparatus that includes an elongate barrel, shaped to define a channel therethrough, in which a porous carrier, that holds a surfactant, is disposed. A sponge is coupled to the distal portion of a plunger that is dimensioned to slide snugly within the channel. While the sponge holds saliva, sliding of the plunger through the channel compresses the sponge within the channel. Compression of the sponge within the channel drives at least a portion of the saliva: (i) out of the sponge and through the carrier, and (ii) from the carrier, together with at least a portion of the surfactant, into a collection tube that is reversibly couplable to an outlet at a distal region of the channel. Other embodiments are also described.

EP 0520408 (A2) discloses a sampling device which can be used to safely obtain samples of biological fluids, such as, for example, saliva, urine, water, tears, and vaginal fluids.

US 2003/064526 (A1) discloses a sample collector and test device which may be used together as a diagnostic tool for collecting and assay of analytes contained in a sample.

US 8025851 (B2) discloses a specimen sample collection device and test system including a handle with a sufficiency indicator and locking teeth, an absorbent pad partially contained within the handle; a pad compression tube with outlet ports and locking holes insertable over the pad and handle; one or more collection tubes attachable to the pad compression tube outlet ports

### SUMMARY OF THE INVENTION

The invention is defined by the independent claims. Preferred embodiments are defined by the dependent claims.

### SUMMARY OF THE DISCLOSURE

Diagnostic assays designed for home- or point-of-care testing are intended to be convenient and accessible to the subject being tested. Ideally, such assays may be reliably performed even by an unskilled user. As provided by some applications of the present disclosure, the reliability of home-testing assays that are performed using a body fluid is increased by standardizing the quantity of body fluid that is collected for testing.

For example, collecting a minimum quantity of body fluid may increase the reliability of the diagnostic assay. Some applications of the present disclosure are therefore directed to determining if a minimum quantity of a body fluid has been collected, without requiring technical expertise or equipment that might overburden the unskilled user.

For some applications, an indicator (e.g., a visual indicator) allows the user to determine if the minimum quantity of body fluid has been collected. For some such applications, controlling flow of the body fluid, through a medium and to the indicator, facilitates the determination of whether the minimum quantity of the body fluid has been collected. If the minimum quantity of the body fluid has been collected, the body fluid may then be passed into a test apparatus for testing.

For some applications of the present disclosure, apparatus described herein may be used with any fluid, not only body fluid, and the indicator may be used to allow the user to determine if the minimum quantity of fluid has been collected. For example, apparatus described herein may be used to determine the purity of water, and the indicator may be used to allow the user to determine if the minimum quantity of water has been collected. For applications in which the apparatus is used with body fluid, the fluid may be saliva, urine, blood, or any other bodily fluid.

For applications in which apparatus described herein may be used with body fluid, it is to be noted that the body fluid can be collected in any suitable noninvasive and nonsurgical manner. For example, as described herein, typically, but not necessarily, the collector of the body fluid is a sponge, which is noninvasive and nonsurgical. Additionally, the body fluid is not returned to the subject after testing. The methods described herein are non-therapeutic and/or non-surgical and do not constitute substantial physical intervention on the body of a living subject.

There is therefore provided, in accordance with a non-claimed application of the present disclosure, a method for use with a body fluid, the method including:
using a collecting tool that includes a collector fixedly coupled to a stem, collecting a body fluid into the collector, the collecting tool defining a pivot-point;
using a fulcrum, supporting the collecting tool at the pivot-point;
while the collecting tool remains supported at the pivot-point, observing an indication of a quantity of the body fluid that is collected into the collector;
responsively to the indication, determining whether a predetermined minimum quantity of the body fluid has been collected into the collector;
if the collector is determined to not have absorbed the predetermined minimum quantity of the body fluid, repeating the step of collecting; and
if the collector is determined to have absorbed the predetermined minimum quantity of the body fluid, testing the body fluid that was collected into the collector.

In an application, the predetermined minimum quantity is 100 - 500 mg.

In an application, the step of testing includes passing the body fluid from the collector to a test apparatus.

In an application, the step of supporting includes suspending the collecting tool at the fulcrum.

In an application, the step of observing includes observing a tilt of the collecting tool.

In an application:
the collecting tool includes an indicator that displays an indication of the tilt of the collecting tool, and
the step of observing includes observing the indication.

In an application:
the indicator displays a moving indication of the tilt of the collecting tool, and
the step of supporting includes supporting the collecting tool at the pivot-point, such that:
   if the collector has absorbed the predetermined minimum quantity of the body fluid, a portion of the indicator moves in a first direction, and
   if the collector has not absorbed the predetermined minimum quantity of the body fluid, the portion of the indicator moves in a second direction.

In an application, the portion of the indicator is a bubble.

In an application, the portion of the indicator is configured to slide or roll responsively to the tilt of the collecting tool.

In an application, the collecting tool includes a docking station at the pivot-point.

In an application, the step of supporting includes suspending the collecting tool at the fulcrum, from the docking station.

In an application, the method includes, prior to the step of suspending, reversibly connecting the fulcrum to the docking station.

In an application, the step of connecting includes magnetically coupling the fulcrum to the docking station.

In an application, the step of connecting includes connecting the fulcrum to the docking station by an interference fit.

In an application, the method includes, subsequently to the step of observing, disconnecting the fulcrum from the docking station.

In an application, the step of testing includes testing the body fluid that was collected into the collector, subsequently to disconnecting the fulcrum from the docking station.

In an application:
the collector includes a sponge, and
collecting the body fluid into the collector includes absorbing the body fluid into the sponge.

In an application, the step of testing includes:
introducing the sponge into a channel, the channel defining an outlet at a distal portion of the channel; and
sliding the sponge distally toward the outlet, thereby:
   compressing the sponge within the channel, driving the body fluid out of the sponge and through the outlet, into a test apparatus.

In an application:
a porous carrier, holding a reagent, is disposed within the channel, and
the step of compressing includes driving the body fluid:
   out of the sponge and through the carrier, dissolving at least some of the reagent, and
   with the dissolved reagent, through the outlet and into the test apparatus.

There is further provided, in accordance with an application of the present disclosure, an apparatus for use with a body fluid, the apparatus including:
a collecting tool, the collecting tool:
   defining a stem and a pivot-point, and
   including a collector fixedly coupled to the stem; and
a fulcrum reversibly couplable to the pivot-point, and
the collecting tool is configured such that while the collecting tool is supported by the fulcrum at the pivot-point, a tilt of the collecting tool indicates a quantity of body fluid collected into the collector.

In an application:
the collecting tool includes an indicator that, while the collecting tool is supported by the fulcrum at the pivot-point, displays an indication of the tilt of the collecting tool.

In an application:
the indicator is configured to display, while the collecting tool is supported by the fulcrum at the pivot-point, a moving indication of the tilt of the collecting tool, such that:
if a predetermined minimum quantity of the body fluid has been collected into the collector, a portion of the indicator moves in a first direction, and
if the predetermined minimum quantity of the body fluid has not been collected into the collector, the portion of the indicator moves in a second direction.

In an application, the portion of the indicator is a bubble.

In an application, the portion of the indicator is configured to slide or roll responsively to the tilt of the collecting tool.

In an application, the collector includes a sponge that is configured to absorb the body fluid.

In an application, the apparatus includes an elongate barrel, the barrel shaped to define a channel therethrough, the channel having:
an opening into the channel at a proximal region of the barrel, and
an outlet from the channel at a distal region of the barrel;
and:
the sponge is fixedly coupled to a distal portion of the collecting tool; and
the collecting tool is:
   configured to introduce the sponge into the channel via the opening, and
   dimensioned such that sliding the distal portion of the collecting tool distally through the channel, while the sponge holds the body fluid, compresses the sponge within the channel, driving the body fluid:
      out of the compressed sponge and through the outlet.

In an application, the apparatus includes a test apparatus, the test apparatus being in fluid communication with the outlet, such that:
sliding the distal portion of the collecting tool distally through the channel, while the sponge holds the body fluid, compresses the sponge within the channel, driving the body fluid:
out of the compressed sponge, through the outlet and into the test apparatus.

In an application, the apparatus includes a porous carrier disposed within the channel, the carrier holding a reagent, such that:
sliding the distal portion of the collecting tool distally through the channel, while the sponge holds the body fluid, compresses the sponge within the channel, driving the body fluid:
out of the sponge and though the carrier, dissolving at least some of the reagent, and
with the dissolved reagent, through the outlet.

In an application, the apparatus includes a test apparatus, the test apparatus being in fluid communication with the outlet, such that:
sliding the distal portion of the collecting tool distally through the channel, while the sponge holds the body fluid, compresses the sponge within the channel, driving the body fluid:
   out of the sponge and though the carrier, dissolving at least some of the reagent, and
with the dissolved reagent, through the outlet and into the test apparatus.

In an application, the collecting tool and the fulcrum are configured such that, while the collecting tool is supported by the fulcrum at the pivot-point, the collecting tool tilts:
in a first direction, if a predetermined minimum quantity of the body fluid has been collected into the collector, or
in a second direction, if the predetermined minimum quantity of the body fluid has not been collected into the collector.

In an application:
the apparatus is configured to have, while the collecting tool is supported by the fulcrum at the pivot-point, a detection sensitivity that represents a minimum difference between:
   a quantity of the body fluid in the collector that will cause the collecting tool to tilt in the first direction, and
   a quantity of the body fluid in the collector that will cause the collecting tool to tilt in the second direction; and
the detection sensitivity is less than 100 mg.

In an application, the detection sensitivity is 5-50 mg.

In an application:
the collecting tool includes a docking station that defines the pivot-point, and
the fulcrum and the docking station are reversibly connectable so as to suspend the collecting tool at the fulcrum.

In an application:
the fulcrum and the docking station each includes a ferromagnetic material, and
the fulcrum and the docking station are reversibly connectable by magnetic attraction between the respective ferromagnetic materials.

In an application, the fulcrum and the docking station are reversibly connectable by an interference fit.

There is further provided, in accordance with a non-claimed application of the present disclosure, a method for use with a body fluid, the method including:
collecting a body fluid into a collector that is fixedly coupled to a collecting tool, the collecting tool including a first ferromagnetic material;
causing the first ferromagnetic material of the collector to interact magnetically with a second ferromagnetic material, such that:
   if a predetermined minimum quantity of the body fluid has been collected into the collector, the collecting tool assumes a first state, and
   if the predetermined minimum quantity of the body fluid has not been collected into the collector, the collecting tool assumes a second state;
observing if the collecting tool is in the first state or if the collecting tool is in the second state;
responsively to the step of observing, determining whether the predetermined minimum quantity of the body fluid has been collected into the collector;
if the collector is determined to not have absorbed the predetermined minimum quantity of the body fluid, repeating the step of collecting; and
if the collector is determined to have absorbed at least the predetermined minimum quantity of the body fluid, testing the body fluid that was collected into the collector.

In an application, the predetermined minimum quantity is 100 - 500 mg.

In an application, the step of testing includes passing the body fluid from the collector to a test apparatus.

In an application, the step of observing includes observing a test-distance between the collecting tool and the second ferromagnetic material.

In an application:
while the collection tool is in the first state, the collection tool is disposed at a first test-distance from the second ferromagnetic material, and
while the collection tool is in the second state, the collection tool is disposed at a second test-distance from the second ferromagnetic material.

In an application:
the collector includes a sponge, and
collecting the body fluid into the collector includes absorbing the body fluid into the sponge.

In an application, the step of testing includes:
introducing the sponge into a channel, the channel defining an outlet at a distal portion of the channel; and
sliding the sponge distally toward the outlet, thereby:
   compressing the sponge within the channel, driving the body fluid out of the sponge and through the outlet, into a test apparatus.

In an application:
a porous carrier, holding a reagent, is disposed within the channel, and
the step of compressing includes driving the body fluid:
   out of the sponge and through the carrier, dissolving at least some of the reagent, and
   with the dissolved reagent, through the outlet and into the test apparatus.

There is further provided, in accordance with a non-claimed application of the present disclosure, apparatus for use with a body fluid, the apparatus including:
a collecting tool including:
   a collector configured for collecting the body fluid, and
   a first ferromagnetic material; and
a second ferromagnetic material,
and:
the second ferromagnetic material is configured to interact magnetically with the first ferromagnetic material, such that:
if a predetermined minimum quantity of the body fluid has been collected into the collector, the collecting tool assumes a first state, and
if the predetermined minimum quantity of the body fluid has not been collected into the collector, the collecting tool assumes a second state.

In an application:
the apparatus is configured to have, while the first ferromagnetic material of the collector interacts magnetically with the second ferromagnetic material, a detection sensitivity that represents a minimum difference between:
   a quantity of the body fluid in the collector that will cause the collecting tool to assume the first state, and
   a quantity of the body fluid in the collector that will cause the collecting tool to assume the second state; and
the detection sensitivity is less than 100 mg.

In an application, the detection sensitivity is 5-50 mg.

In an application, the apparatus is configured such that:
while the collection tool is in the first state, the collection tool is disposed at a first test-distance from the second ferromagnetic material, and
while the collection tool is in the second state, the collection tool is disposed at a second test-distance from the second ferromagnetic material.

In an application, the collector includes a sponge that is configured to absorb the body fluid.

In an application, the apparatus includes an elongate barrel, the barrel shaped to define a channel therethrough, the channel having:
an opening into the channel at a proximal region of the barrel, and
an outlet from the channel at a distal region of the barrel;
and:
the sponge is fixedly coupled to a distal portion of the collecting tool; and
the collecting tool is:
   configured to introduce the sponge into the channel via the opening, and
   dimensioned such that sliding the distal portion of the collecting tool distally through the channel, while the sponge holds the body fluid, compresses the sponge within the channel, driving the body fluid:
      out of the compressed sponge and through the outlet.

In an application, the apparatus includes a test apparatus, the test apparatus being in fluid communication with the outlet, such that:
sliding the distal portion of the collecting tool distally through the channel, while the sponge holds the body fluid, compresses the sponge within the channel, driving the body fluid:
out of the compressed sponge, through the outlet and into the test apparatus.

In an application, the apparatus includes a porous carrier disposed within the channel, the carrier holding a reagent, such that:
sliding the distal portion of the collecting tool distally through the channel, while the sponge holds the body fluid, compresses the sponge within the channel, driving the body fluid:
out of the sponge and though the carrier, dissolving at least some of the reagent, and
with the dissolved reagent, through the outlet.

In an application, the apparatus includes a test apparatus, the test apparatus being in fluid communication with the outlet, such that:
sliding the distal portion of the collecting tool distally through the channel, while the sponge holds the body fluid, compresses the sponge within the channel, driving the body fluid:
   out of the sponge and though the carrier, dissolving at least some of the reagent, and
with the dissolved reagent, through the outlet and into the test apparatus.

There is additionally provided, in accordance with an application of the disclosure, apparatus for use with a body fluid, the apparatus including:
a collector configured to absorb the body fluid;
an indicator configured to indicate that at least some of the body fluid has reached the indicator; and
a medium, the medium:
   providing fluid communication between the collector and the indicator, so as to facilitate, while the collector holds the body fluid, passage of some of the body fluid from the collector, through the medium, to the indicator, and
   having a flow resistance, per cubic millimeter of the medium, that is greater than a flow resistance per cubic millimeter of the collector.

In an application, a dry volume of at least 50 cubic millimeters of the medium separates the collector from the indicator.

In an application, a dry volume of less than 100 cubic millimeters of the medium separates the collector from the indicator.

In an application, the medium is configured to absorb up to 20 microliters of body fluid.

In an application, the collector is configured to absorb at least 100 microliters of body fluid.

In an application, the collector is configured to absorb up to 900 microliters of body fluid.

In an application, the apparatus further includes a restraining tube:
at least a portion of the medium is housed within a part of the restraining tube, such that the medium facilitates passage of some of the body fluid from the collector, through the portion of the medium, to the indicator.

In an application, for a predetermined test period of 60 seconds, the apparatus is configured to facilitate, while the collector holds the body fluid, passage of less than 20 microliters of the body fluid from the collector, through the portion of the medium and to the indicator, during the predetermined test period.

In an application, the medium and the restraining tube are configured such that the some of the body fluid requires at least 30 seconds to pass from the collector to the indicator.

In an application, the part of the restraining tube has an internal width that is between 2 and 10 mm.

In an application, the medium has a dry width and an unconstrained saturated width, and the unconstrained saturated width of the portion of the medium is greater than the internal width of the part of the restraining tube.

In an application, the part of the restraining tube has an internal volume of 200 - 1000 cubic millimeters.

In an application, the medium has a dry volume and an unconstrained saturated volume, and the unconstrained saturated volume of the portion of the medium is greater than the internal volume of the part of the restraining tube.

In an application, a length of at least 10 mm of the medium separates the collector from the indicator.

In an application, a length of at most 50 mm of the medium separates the collector from the indicator.

In an application, a length of at most 10 cm of the medium separates the collector from the indicator.

In an application, the medium includes cotton and regenerated cellulose.

In an application, the medium includes, by mass, more regenerated cellulose than cotton.

In an application, the medium includes, by mass, at least 1.5 times more regenerated cellulose than cotton.

In an application, configured such that, during a predetermined test period of 60 seconds:
if the collector holds a minimum test-quantity of the body fluid, at least a minimum indication-quantity of body fluid will flow from the collector, through the medium, to the indicator, and
if the collector does not hold the minimum test-quantity of the body fluid, the minimum indication-quantity of body fluid will not flow from the collector, through the medium and to the indicator.

In an application, the apparatus includes a restraining tube:
at least a portion of the medium is housed within a part of the restraining tube, such that the medium facilitates passage of some of the body fluid from the collector, through the portion of the medium, to the indicator;
the medium has a dry state and a saturated state;
the medium and the restraining tube are configured such that, while the body fluid passes from the collector, through the portion of the medium and to the indicator:
   the medium transitions from the dry state to the saturated state, and
   the restraining tube inhibits radial expansion of the medium by mechanically restraining the medium; and
for a predetermined test period of 60 seconds, the apparatus is configured to facilitate, while the collector holds the body fluid, passage of less than 20 microliters of the body fluid from the collector, through the medium and to the indicator, during the predetermined test period.

In an application, the minimum test-quantity is 350 microliters, and the minimum indication-quantity is less than 10 microliters.

In an application, the minimum indication-quantity less than 2 microliters.

In an application, the collector is configured to absorb a collector-capacity of at least 100 microliters of body fluid.

In an application, the collector-capacity is less than 1000 microliters of body fluid.

In an application, the minimum test-quantity is less than ten percent of the collector-capacity.

In an application, the minimum test-quantity is less than one percent of the collector-capacity.

There is further provided, in accordance with a non-claimed application of the disclosure, a method for use with a body fluid, the method including:
absorbing the body fluid into a collector;
allowing a portion of the body fluid to pass from the collector and through a medium toward an indicator, the medium having a flow resistance, per cubic millimeter of the medium, that is greater than a flow resistance per cubic millimeter of the collector;
observing that the indicator has provided an indication that at least some of the body fluid has reached the indicator; and
responsively to observing that the indicator has provided the indication, extracting the body fluid from the collector.

In an application:
the step of absorbing includes, using the collector, absorbing the body fluid from a body of a subject, and
the method does not include using the medium to absorb the body fluid from the body of the subject.

In an application, the step of absorbing includes absorbing 100-900 microliters of body fluid into the collector.

In an application, the step of extracting including extracting 100-900 microliters of the body fluid from the collector.

In an application, the step of observing includes observing that the indicator has provided an indication that at least 0.5 microliters of the body fluid have reached the indicator.

In an application, the method further includes, prior to observing that the indicator has provided the indication that at least some of the body fluid has reached the indicator:
observing that the indicator has not provided the indication that at least some of the body fluid has reached the indicator, and
repeating the step of absorbing.

In an application, the method does not include extracting the body fluid from the indicator.

In an application, the method does not include extracting the body fluid from the medium.

In an application, the step of allowing the portion of the body fluid to pass includes, during a predetermined test period, allowing the portion of the body fluid to pass from the collector and through the medium toward the indicator.

In an application, the predetermined testing period is 30 - 60 seconds.

In an application, the step of observing includes, during the predetermined testing period, observing that the indicator has provided the indication that at least some of the body fluid has reached the indicator.

In an application, the step of allowing the portion of the body fluid to pass includes, during the predetermined test period, allowing the portion of the body fluid to pass from the collector and through a length of at least 10 mm of the medium toward the indicator.

In an application, the step of allowing the portion of the body fluid to pass includes, during the predetermined test period, allowing the portion of the body fluid to pass from the collector and through a volume of at least 100 cubic millimeters of the medium toward the indicator.

In an application, the step of allowing the portion of the body fluid to pass includes, during the predetermined test period, allowing less than ten percent of the absorbed body fluid to pass from the collector and through the medium toward the indicator.

In an application, the step of allowing the portion of the body fluid to pass includes, during the predetermined test period, allowing less than one percent of the absorbed body fluid to pass from the collector and through the medium toward the indicator.

In an application, the step of allowing the portion of the body fluid to pass includes, during the predetermined test period, allowing less than 20 microliters of the body fluid to pass from the collector and through the medium toward the indicator.

In an application, the step of allowing the portion of the body fluid to pass includes, during the predetermined test period, allowing less than 2 microliters of the body fluid to pass from the collector and through the medium toward the indicator.

In an application:
the medium is at least partially housed within a restraining tube,
the medium has a dry state and a saturated state,
the step of allowing the body fluid to pass from the collector and through the medium toward the indicator includes causing the medium to transition from the dry state to the saturated state,
the method further includes, while the medium transitions from the dry state to the saturated state, using the restraining tube to inhibit expansion of the medium by mechanically restraining the medium.

There is additionally provided, in accordance with a non-claimed application of the disclosure, a method for use with a fluid, the method including:
using a collecting tool that includes a collector fixedly coupled to a stem, collecting the fluid into the collector, the collecting tool defining a pivot-point;
using a fulcrum, supporting the collecting tool at the pivot-point;
while the collecting tool remains supported at the pivot-point, observing an indication of a quantity of the fluid that is collected into the collector;
responsively to the indication, determining whether a predetermined minimum quantity of the fluid has been collected into the collector;
if the collector is determined to not have absorbed the predetermined minimum quantity of the fluid, repeating the step of collecting; and
if the collector is determined to have absorbed the predetermined minimum quantity of the fluid, testing the fluid that was collected into the collector.

There is yet further provided, in accordance with an application of the disclosure, apparatus for use with a fluid, the apparatus including:
a collecting tool, the collecting tool:
   defining a stem and a pivot-point, and
   including a collector fixedly coupled to the stem; and
a fulcrum reversibly couplable to the pivot-point,
the collecting tool is configured such that while the collecting tool is supported by the fulcrum at the pivot-point, a tilt of the collecting tool indicates a quantity of the fluid collected into the collector.

There is yet additionally provided, in accordance with a non-claimed application of the disclosure, a method for use with a fluid, the method including:
collecting the fluid into a collector that is fixedly coupled to a collecting tool, the collecting tool including a first ferromagnetic material;
causing the first ferromagnetic material of the collector to interact magnetically with a second ferromagnetic material, such that:
   if a predetermined minimum quantity of the fluid has been collected into the collector, the collecting tool assumes a first state, and
   if the predetermined minimum quantity of the fluid has not been collected into the collector, the collecting tool assumes a second state;
observing if the collecting tool is in the first state or if the collecting tool is in the second state;
responsively to the step of observing, determining whether the predetermined minimum quantity of the fluid has been collected into the collector;
if the collector is determined to not have absorbed the predetermined minimum quantity of the fluid, repeating the step of collecting; and
if the collector is determined to have absorbed at least the predetermined minimum quantity of the fluid, testing the fluid that was collected into the collector.

There is also provided, in accordance with a non-claimed application of the disclosure, apparatus for use with a fluid, the apparatus including:
a collecting tool including:
   a collector configured for collecting the fluid, and
   a first ferromagnetic material; and
a second ferromagnetic material,
the second ferromagnetic material is configured to interact magnetically with the first ferromagnetic material, such that:
   if a predetermined minimum quantity of the fluid has been collected into the collector, the collecting tool assumes a first state, and
   if the predetermined minimum quantity of the fluid has not been collected into the collector, the collecting tool assumes a second state.

There is additionally provided, in accordance with a non-claimed application of the disclosure, apparatus for use with a fluid, the apparatus including:
a collector configured to absorb the fluid;
an indicator configured to indicate that at least some of the fluid has reached the indicator; and
a medium, the medium:
   providing fluid communication between the collector and the indicator, so as to facilitate, while the collector holds the fluid, passage of some of the fluid from the collector, through the medium, to the indicator, and
   having a flow resistance, per cubic millimeter of the medium, that is greater than a flow resistance per cubic millimeter of the collector.

There is also provided, in accordance with a non-claimed application of the disclosure, a method for use with a fluid, the method including:
absorbing the fluid into a collector;
allowing a portion of the fluid to pass from the collector and through a medium toward an indicator, the medium having a flow resistance, per cubic millimeter of the medium, that is greater than a flow resistance per cubic millimeter of the collector;
observing that the indicator has provided an indication that at least some of the fluid has reached the indicator; and
responsively to observing that the indicator has provided the indication, extracting the fluid from the collector.

The present disclosure will be more fully understood from the following detailed description of applications thereof, taken together with the drawings, in which:

### BRIEF DESCRIPTION OF THE DRAWINGS

Figs. 1, 2A-C, 3A-B, 4A-B, 5A-B, 6A-B and 7A-B are schematic illustrations showing systems for use with a body fluid, in accordance with some applications of the present disclosure;
Figs. 8A-B are schematic illustrations showing a non-claimed system for use with body fluid, in accordance with some applications of the disclosure;
Figs. 9A-C and 10A-B are schematic illustrations showing non-claimed systems for use with body fluid, in accordance with some applications of the disclosure;
Figs. 11-12 are flowcharts that schematically illustrate at least some steps of non-claimed methods that are performed in accordance with some applications of the present disclosure;
Fig. 13 shows schematic illustrations of a non-claimed indicator assembly, in accordance with some applications of the disclosure;
Figs. 14A-B are schematic illustrations showing a non-claimed collecting tool comprising the indicator assembly, in accordance with some applications of the disclosure;
Figs. 15A-C and 16A-B are schematic illustrations showing non-claimed systems for use with body fluid, in accordance with some applications of the disclosure; and
Figs. 17-18 are flowcharts that schematically illustrate at least some steps of non-claimed methods that are performed in accordance with some applications of the present disclosure.

### DETAILED DESCRIPTION OF EMBODIMENTS

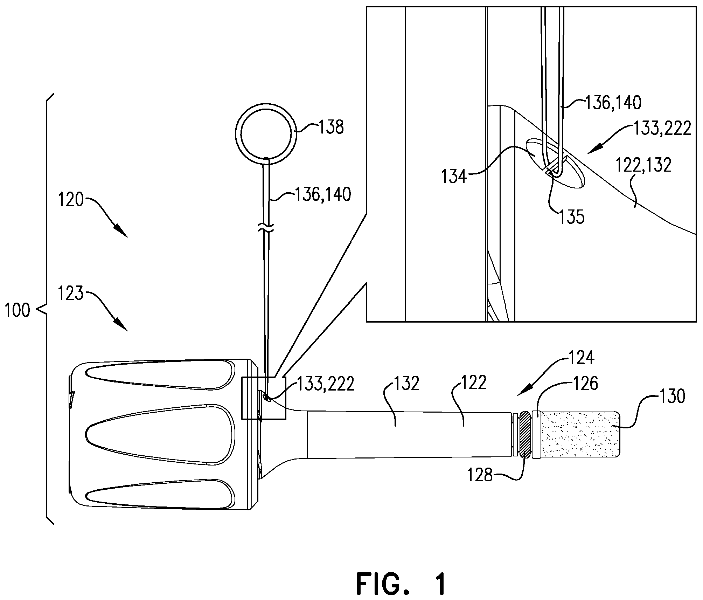

Reference is made to Figs. 1, 2A-C, 3A-B, 4A-B, 5A-B, 6A-B and 7A-B, which are schematic illustrations showing systems 100, 200, 600, 700, 800 and 900 for use with a body fluid 150, in accordance with some applications of the disclosure.

As shown in Fig. 1, system 100 comprises a collecting tool 120 that defines a stem 132 and a collector 130 (e.g., an absorbent sponge, as shown) for collecting a quantity of body fluid. For some applications, and as shown, collector 130 is fixedly coupled to stem 132, e.g., to a plunger 122 formed by the stem, at a distal portion 124 of tool 120. For some such applications, and as shown, collector 130 is coupled to a portion of tool 120 that is distal of a crown 126 and piston ring 128, as described hereinbelow.

Typically and as shown, tool 120 defines a pivot-point 133, e.g., at a hole 134, that is defined by the tool between a proximal portion 123 and distal portion 124. Further typically, system 100 comprises a fulcrum 140 (e.g., a string 136) that may be reversibly coupled to pivot-point 133. In this way, tool 120 defines a docking station 222 at which fulcrum 140 may be reversibly coupled to pivot-point 133. For some applications, and as shown, string 136 is coupled to docking station 222 by passing through hole 134, around a bar 135 that is defined by tool 120. For some such applications, string 136 is also coupled to a ring 138 or other type of handle, for ease of use.

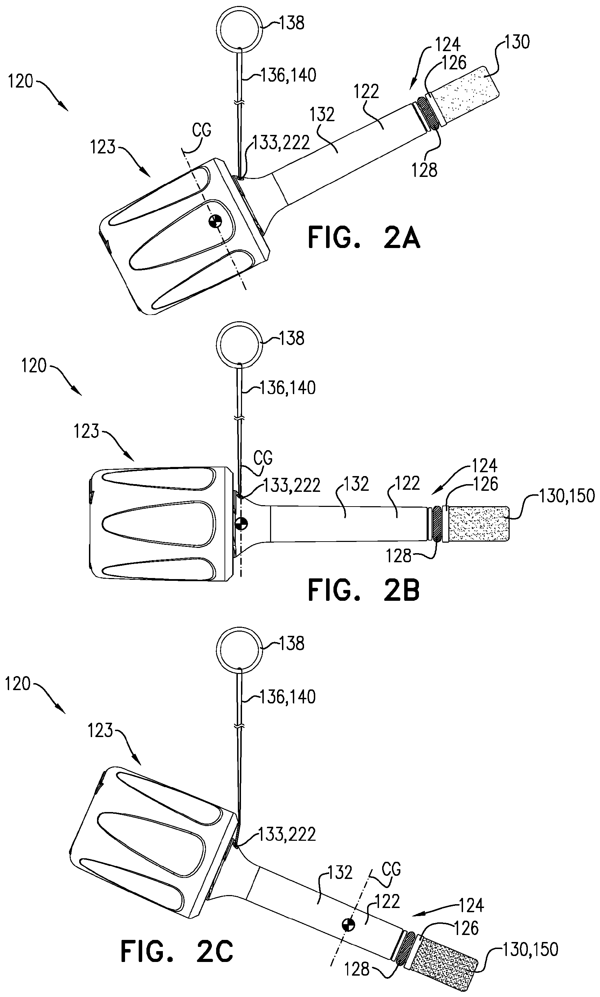

In this way, when tool 120 is supported at pivot-point 133, the tool may tilt, as dictated by the relative weights of proximal portion 123 of the tool and distal portion 124 of the tool. Figs. 2A-C shows tool 120 at three different states of tilt while string 136 supports tool 120 at pivot-point 133.

Fig. 2A shows tool 120 tilted with distal portion 124 elevated in relation to proximal portion 123, with the tool's center of gravity located proximally of pivot-point 133. For some applications, tool 120 will assume this tilt if collector 130 has not collected a predetermined minimum quantity (e.g., 100 - 500 mg) of body fluid, such that proximal portion 123 is heavier than distal portion 124. For example, the location of pivot-point 133 may be calibrated in relation to the relative weights of proximal portion 123 and distal portion 124, so that the distal portion will be elevated if the distal portion lacks additional weight of the predetermined minimum quantity of the body fluid.

Fig. 2B shows tool 120 at equilibrium, in which proximal portion 123 and distal portion 124 are at a similar height. Collector 130 is shown with somewhat darker shading than in Fig. 2A, indicating that a certain amount (e.g., less than the minimum quantity) of body fluid has been absorbed into the sponge. As shown, the additional weight of the collected body fluid is sufficient to make the weight of distal portion 124 equivalent to the weight of proximal portion 123, such that the tool's center of gravity passes through pivot-point 133.

Fig. 2C shows tool 120 tilted with proximal portion 123 elevated in relation to distal portion 124, with the tool's center of gravity located distally of pivot-point 133. For some applications, tool 120 will assume this tilt if collector 130 has collected the minimum quantity of body fluid, such that distal portion 124 is heavier than proximal portion 123.

In this way, the tilt that tool 120 assumes while supported at pivot-point 133 provides an easily observable indication of whether the minimum quantity of body fluid has been collected into collector 130. It is typically not necessary for the user to know the exact quantity of body fluid collected, as long as the minimum quantity has been collected. The inventors have performed experiments demonstrating that a detection sensitivity of system 100 (e.g., a difference in the quantity of body fluid 150 held in collector 130 that will determine whether tool 120 will tilt with distal portion 124 or proximal portion 123 elevated) is less than 100 mg, such as between 5-50 mg.

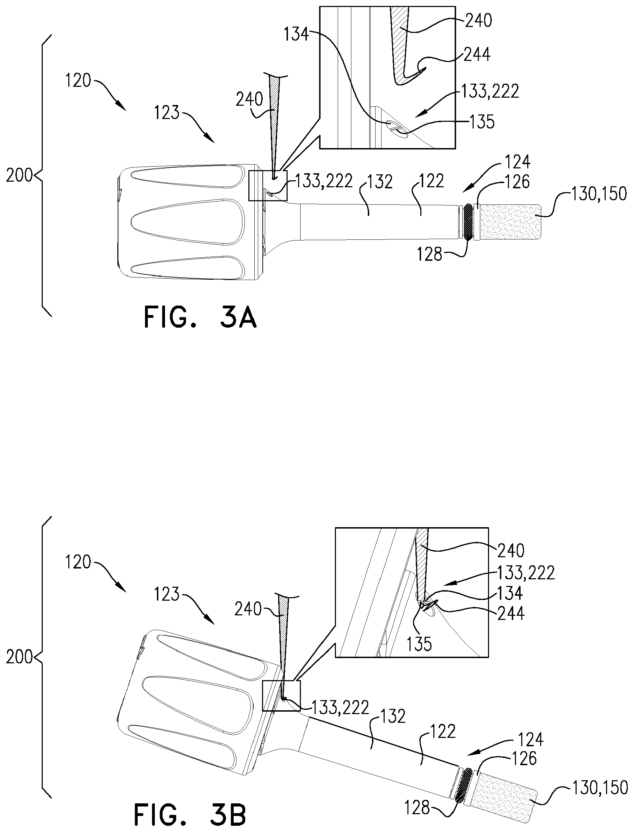

Figs. 3A-B show system 200, which comprises tool 120 and a fulcrum 240. Similarly to fulcrum 140 described hereinabove, fulcrum 240 is reversibly coupled to docking station 222 at pivot-point 133. However, fulcrum 240 is shaped to define a hook 244 that is reversibly coupled to bar 135. Collector 130 contains at least the minimum quantity of body fluid 150, which becomes evident when tool 120 is suspended at pivot-point 133 from hook 244 (Fig. 3B).

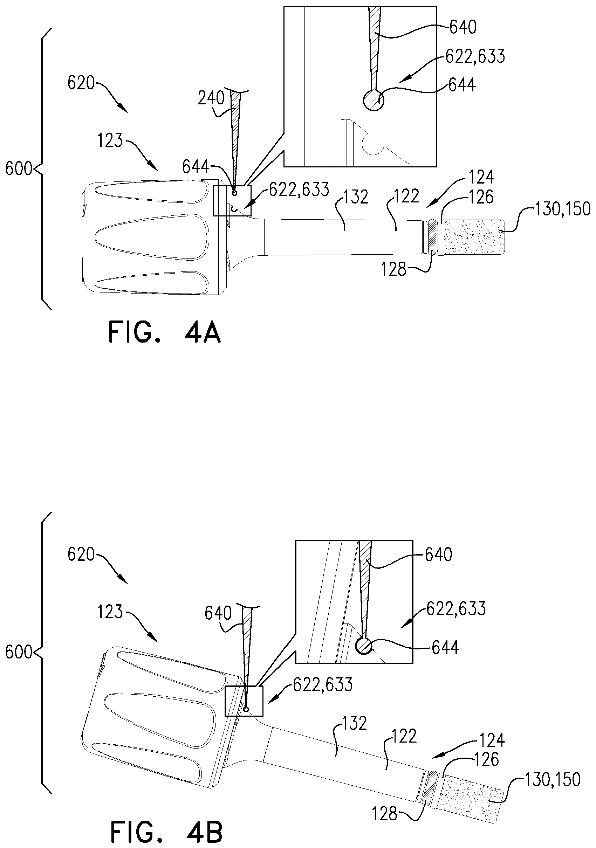

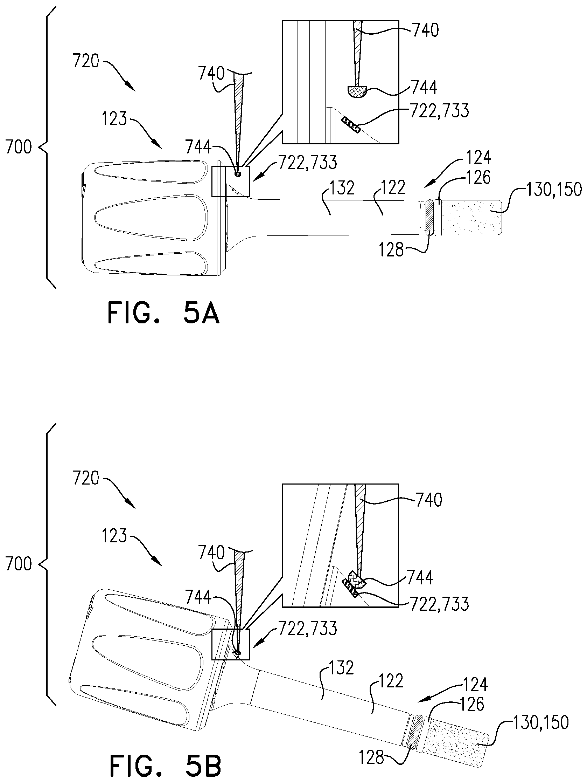

Figs. 4A-B and 5A-B show systems 600 and 700, which respectively comprise a collecting tool 620, 720 and a fulcrum 640, 740. Tools 620, 720 are generally identical to tool 120, except for respective docking stations 622, 722. Similarly to docking station 222 of tool 120, docking stations 622, 722 are used to reversibly couple respective fulcrums 640, 740 to pivot-points 633, 733. However, the mechanisms by which docking stations 622, 722 reversibly couple to fulcrums 640, 740 differ from that by which docking station 222 couples to fulcrums 140, 240.

For some applications, and as shown in Figs. 4A-B, fulcrum 640 is shaped to be reversibly coupled to docking station 622, e.g., by an interference fit, e.g., by an interference fit between a ball 644 that is defined by the fulcrum (or by the docking station), and an indentation that is defined by the docking station (or by the fulcrum). For some applications, and as shown in Figs. 5A-B, fulcrum 740 and docking station 722 each comprise a ferromagnetic material. Typically for such applications, at least one of the ferromagnetic materials is a magnet, such that ferromagnetic material 744 of fulcrum 740 and the ferromagnetic material of docking station 722 are reversibly connectable by magnetic attraction (Fig. 5B). As shown in Figs. 4B and 5B, collector 130 contains at least the minimum quantity of body fluid 150, which becomes evident when tool 620, 720 is suspended at docking station 622, 722 from fulcrum 640, 740.

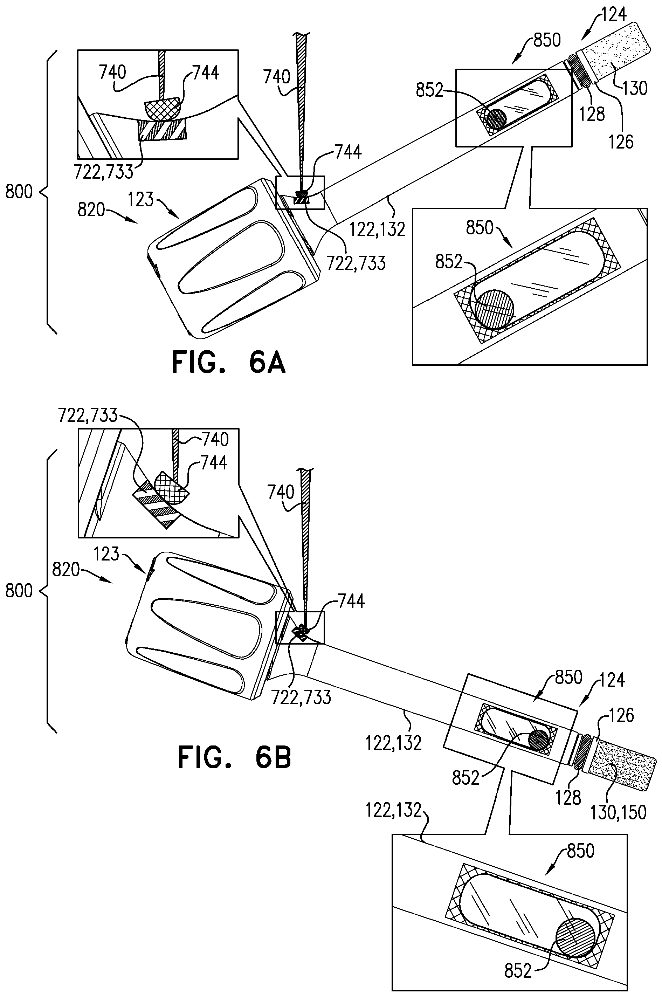

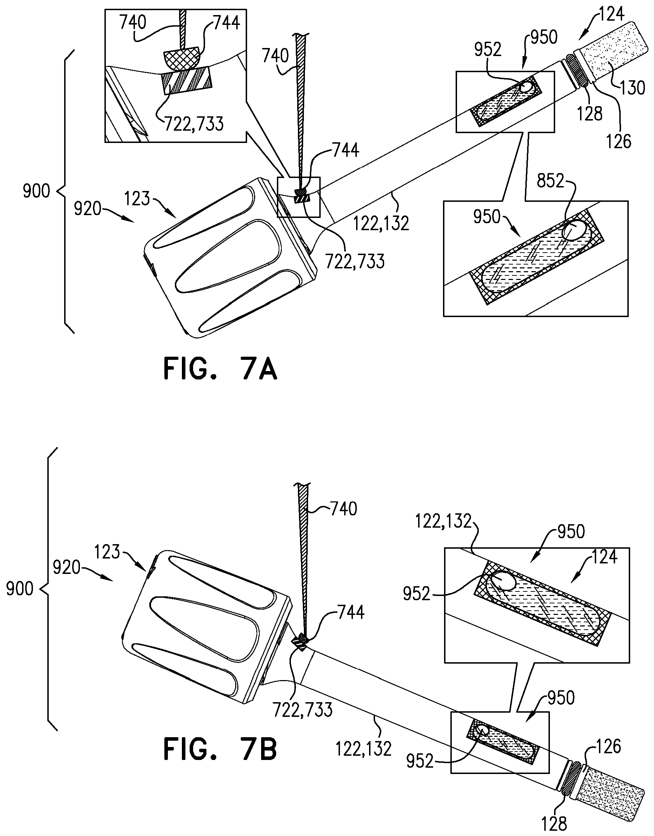

Figs. 6A-B and 7A-B show systems 800 and 900, which each comprise a collecting tool 820, 920 and a fulcrum (e.g., fulcrum 740, as shown). Collection tools 820, 920 are shown being generally identical to tool 720, except that tools 820, 920 additionally comprise an indicator 850, 950, e.g., on stem 132. Other collection tools (e.g., tools 120 and 620 described hereinabove) may also comprise an indicator, *mutatis mutandis.*

As shown, tools 820, 920 are each suspended at pivot-point 733 from fulcrum 740. As shown, while no body fluid is in collector 130, each collection tool tilts with distal portion 124 elevated with respect to proximal portion 123 (Figs. 6A and 7A). After the minimum quantity of body fluid has been collected into collector 130, each tool 820, 920 tilts with proximal portion 123 elevated with respect to distal portion 124 (Figs. 6B and 7B).

Indicators 850, 950 each display an indication of the respective collecting tool's tilt. For some applications, and as shown, the indicator (e.g., a ball 852 of indicator 850, or a bubble 952 of indicator 950) provides a moving indication of the tool's tilt. That is, in response to the tilt at which tool 820 is suspended from pivot-point 733, ball 852 may slide or roll distally or proximally, depending upon whether distal portion 124 or proximal portion 123 of the collection tool is heavier. Similarly, in response to the tilt at which tool 920 is suspended from pivot-point 733, bubble 952 will float toward either distal portion 124 or proximal portion 123, depending upon which portion of the collection tool is lighter. In this way, each indicator 850, 950 displays a readably detectable indication of whether the minimum quantity of body fluid has been collected into collector 130.

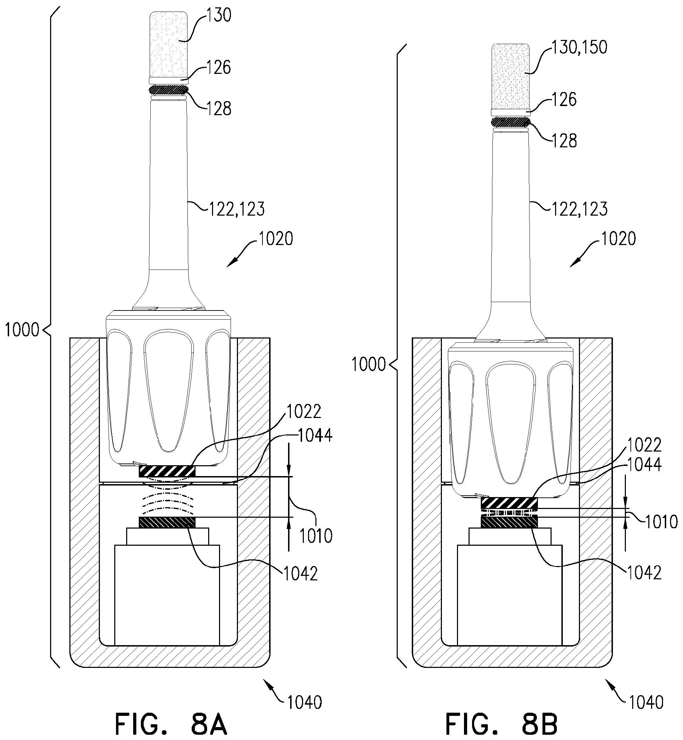

Reference is made to Figs. 8A-B, which are schematic illustrations showing a system 1000 for use with body fluid 150, in accordance with some applications of the disclosure. As shown, system 1000 comprises a collecting tool 1020 that is generally identical to tools 120, 620 and 720, with the exception that instead of a hole or a docking station as described, tool 1020 comprises a ferromagnetic material 1022. System 1000 further comprises a tester 1040 that also has a ferromagnetic material 1042. Typically, at least one of the ferromagnetic materials is a magnet, such that the ferromagnetic materials can interact magnetically to demonstrate whether the minimum quantity of body fluid 150 has been collected into collector 130.

For some applications, and as shown, system 1000 uses magnetic repulsion between the ferromagnetic materials 1022, 1042 to demonstrate whether the minimum quantity of body fluid 150 has been collected. Typically for such applications, system 1000 is calibrated such that if the minimum quantity of body fluid has not been collected into collector 130, collecting tool 1020 will assume a first state (Fig. 8A) in which a test-distance 1010 between the tool and a portion of tester 1040 (e.g., ferromagnetic material 1042 thereof) is greater than when the tool is in a second state (Fig. 8B). As shown in Fig. 8A, collector 130 does not hold body fluid while tool 1020 is in the first state. However, when collector 130 holds at least the minimum quantity of body fluid 150, the additional weight of the body fluid causes gravity to overcome the magnetic repulsion between ferromagnetic materials 1022, 1042. Fig. 8B therefore shows tool 1020 in a second state in which test-distance 1010 has decreased.

The tool's transition from the first state to the second state is typically readily detectable, such that the unskilled observer may easily determine if the minimum quantity of body fluid 150 has been collected. For example, ferromagnetic materials 1022 and 1042 may contact each other while tool 1020 is in the second state. Alternatively or in addition, tester 1040 and/or tool 1020 may include an indicator (e.g., indicator line 1044) that serves as a reference point for tracking motion of tool 1020 as the tool transitions from the first state to the second state.

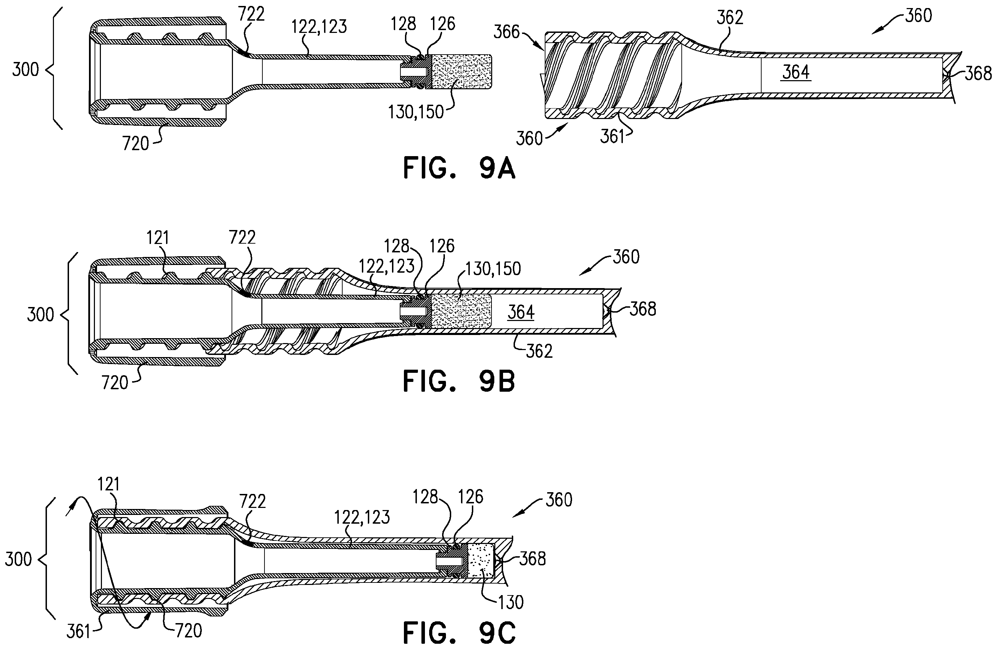

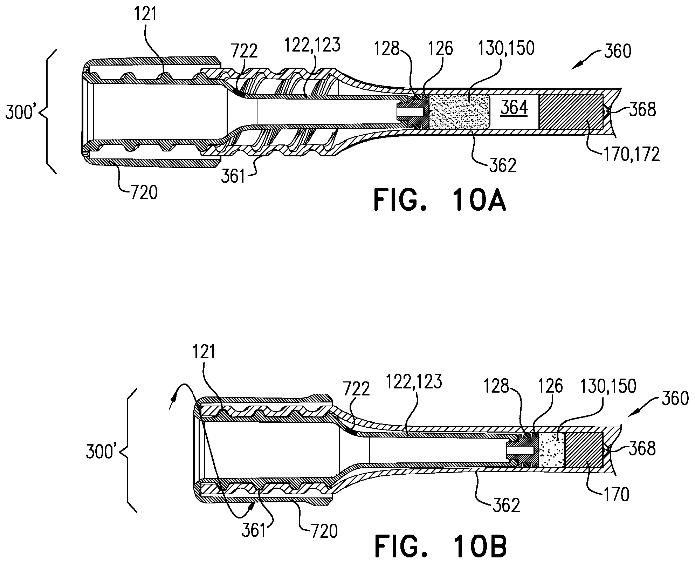

Reference is made to Figs. 9A-C and 10A-B, which are schematic illustrations showing systems 300, 300' for use with body fluid 150, in accordance with some applications of the disclosure.

Typically, the collected body fluid is passed from collector 130 to a test apparatus in order to determine a condition of the subject from whom the body fluid was collected. It is typically desirable to extract the body fluid from collector 130 in a manner that is both efficient (e.g., with a high yield, in terms of the volume of extracted body fluid) and safe (e.g., avoiding contamination of individuals or materials that share the environment in which the body fluid is extracted from the collector).

Thus, Figs. 9A-C and 10A-B illustrate use of systems 300, 300' for extracting body fluid 150 from collector 130, generally in accordance with procedures disclosed in WO 2021/250672 referred to hereinabove. By way of illustration, Figs. 9A-C and 10A-B show extraction of body fluid 150 from collector 130 of tool 720. Similar procedures may be used for extraction of body fluid 150 from collector 130 of tools 120, 620, 820, 920, 1020, *mutatis mutandis.*

Fig. 9A shows tool 720 positioned with distal portion 724 facing a proximal opening 366 of an extractor 360. Typically, and as shown, extractor 360 comprises an elongate barrel 362 that defines a channel 364 therethrough, from opening 366 to a distal outlet 368.

Fig. 9B shows tool 720 having been advanced distally toward extractor 360, thereby introducing collector 130 into channel 364 via opening 366. As shown in Fig. 9C, further sliding of distal portion 724 through channel 364 compresses collector 130 within the channel. Typically, compressing collector 130 drives body fluid 150 (e.g., some or all of the body fluid) out of the compressed sponge and through outlet 368 (e.g., into a test apparatus or a collection tube that is in fluid communication with the outlet). For some applications, and as shown, extractor 360 has a threaded portion 361 that engages threading 121 of tool 720 as the user applies a rotational force (Figs. 9C, 10C) to the tool, thereby advancing and/or compressing collector 130 within channel 364.

Figs 10A-B illustrate use of system 300' to extract body fluid 150 from collector 130 similarly to as described hereinabove with reference system 300 in Figs. 9A-C, with the exception that a porous carrier 170 is disposed within channel 364. Carrier 170 typically holds a reagent, e.g., a surfactant, and/or a protein such as albumin. In this way, compressing collector 130 within channel 364 drives body fluid 150: (i) out of the sponge and though carrier 170, dissolving at least some reagent 172, and (ii) with the dissolved reagent, through outlet 368 and into a test apparatus that is in fluid communication with the outlet.

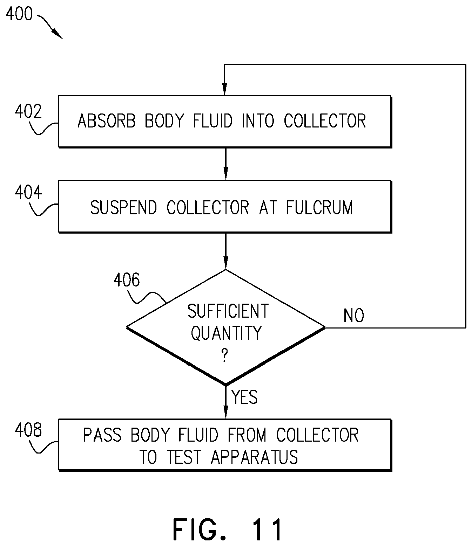

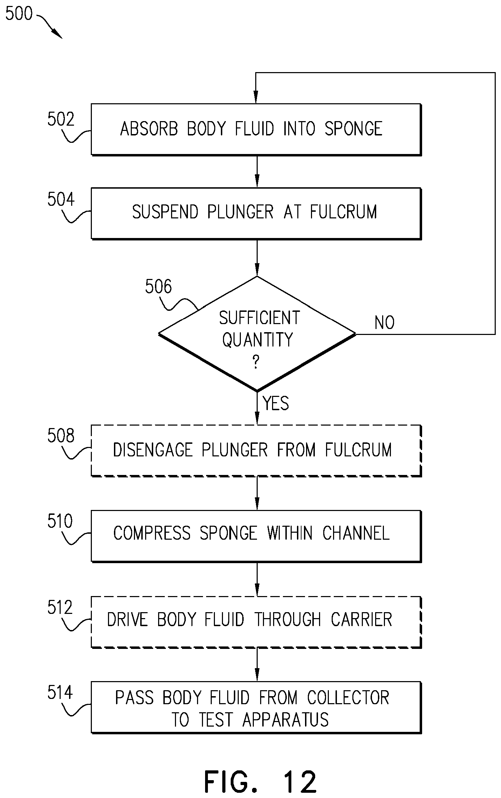

Reference is made to Figs. 11-12, which are flowcharts that schematically illustrate at least some steps of methods 400, 500 that are performed in accordance with some applications of the present disclosure.

As described hereinabove, body fluid 150 is typically absorbed into a collector, such as collector 130 (steps 402, 502), after which the collector is suspended (e.g., by a collecting tool, such as plunger 122) at a fulcrum 140, 240, 640, 740 (steps 404, 504, Figs. 1, 2A-C, 3B, 4B, 5B, 6A-B, 7A-B). A tilt of the tool is observed, in order to determine whether a predetermined minimum quantity of body fluid 150 has been collected into collector 130 (steps 406, 506). For some applications, if collector 130 is determined to not have absorbed the minimum quantity, additional body fluid is collected into the collector (steps 402, 502), after which the collector is again suspended at fulcrum 140, 240, 640, 740 (steps 404, 504).

If the predetermined minimum quantity of body fluid 150 is determined to have been collected into collector 130 (steps 406, 506), the body fluid is typically passed into a test apparatus (steps 408, 514) for testing, e.g., after disconnecting the collecting tool from the fulcrum (step 508). For some applications, collector 130 is compressed within a channel 364 (step 510, Figs. 9B-C, 10A-B), driving body fluid 150 through an outlet 368 of the channel and into the test apparatus. For some such applications, a carrier 170 holding a reagent 172 is disposed within channel 364, such that compressing the sponge drives body fluid 150 through the carrier (step 512), and with dissolved reagent, from outlet 368 and into the test apparatus (step 514). Alternatively or in addition, body fluid 150 may be passed through outlet 368 and into a collection tube for subsequent testing.

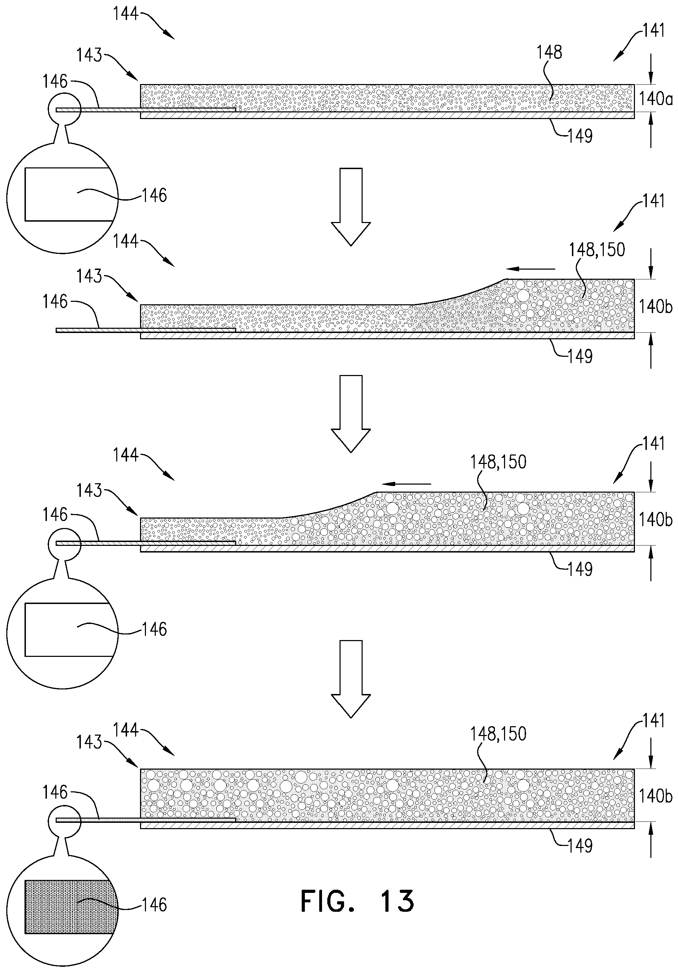

Thus, Figs. 1, 2A-C, 3A-B, 4A-B, 5A-B, 6A-B, 7A-B, 8A-B, 9A-C, 10A-B and 11-12 relate to collecting tools that have either fulcrums or ferromagnetic materials that are used to determine whether collector 130 holds a minimum quantity of body fluid 150. As described in detail hereinbelow, Figs. 13, 14A-B, 15A-C, 16A-B and 17-18 relate to additional apparatus and methods for determining that collector 130 holds the minimum quantity of body fluid 150.

Reference is made to Fig. 13, which shows schematic illustrations of an indicator assembly 144, in accordance with some applications of the disclosure. As shown, assembly 144 comprises indicator 146 that indicates (e.g., by a visual indication, such as a change in color) to the user that body fluid has reached the indicator. Indicator 146 is typically highly sensitive to the presence of body fluid, such that the indicator will indicate that even a minimum indication-quantity of less than 10 microliters, e.g., less than 2 microliters, such as less than 0.5 microliters of body fluid has reached the indicator. Indicator 146 may comprise, for example, SOMA Oral Fluid Collector II (TM), available from Soma Bioscience (TM).

As shown, assembly 144 also comprises medium 148 that is in fluid communication with indicator 146, e.g., at an indicator-end 143 of the medium. Medium 148 typically comprises one or more hydrophilic materials, such that body fluid that is applied to an application-portion 141 of the medium passes along the length of the medium toward indicator-end 143. For some applications, the hydrophilic materials of medium 148 include cotton and/or regenerated cellulose. For some such applications, medium 148 comprises more (e.g., 1.5 times more) regenerated cellulose by mass than cotton. For example, medium 148 may comprise a commercially available absorbent, hydrophilic material, such as Sugi (TM) substrate manufactured by QUESTALPHA GmbH (TM). Typically, since regenerated cellulose moderates the absorption of the cotton, the higher the ratio of regenerated cellulose to cotton, the faster fluid passes along medium 148. For example, medium 148 may comprise a commercially available liquid contact indicator.

As shown in Fig. 13, body fluid 150 that is applied to application-portion 141 of medium 148 passes through the medium toward indicator 146. Frame A of Fig. 13 shows medium 148 in a dry state, prior to application of body fluid to the medium. Frame B of Fig. 13 shows assembly 144 shortly (e.g., a few seconds) after application of body fluid 150 to application-portion 141 of medium 148. As shown in frames B-D of Fig. 13, while the body fluid passes distally along the length of medium 148, progressively distal portions of the medium transition from a dry state to a saturated state. For some applications, and as shown, medium 148 has an unconstrained saturated width 140b that is greater (e.g., approximately two to three times greater) than the medium's dry width 140a.

For some applications, and as shown in Fig. 13, indicator 146 indicates the presence of moisture by a change in color. As shown in the frames A and C, indicator 146 has a first color, as long as indicator-end 143 of medium 148 is dry. As shown in frame D, body fluid 150 that reaches indicator 146 indicator-end 143 of medium 148 causes the indicator to change color. For some applications, indicator 146 comprises a commercially available moisture-sensitive pigment.

For some applications, and as shown, assembly 144 comprises a base 149 that provides mechanical support to the assembly.

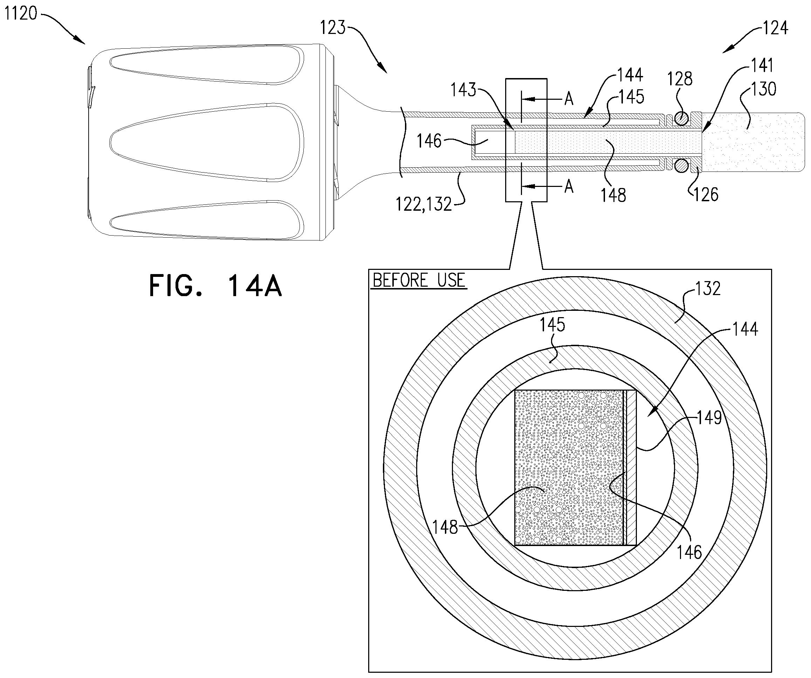

Reference is made to Figs. 14A-B, which are schematic illustrations showing a collecting tool 1120 comprising indicator assembly 144, in accordance with some applications of the disclosure.

As shown, collecting tool 1120 is generally identical to tools 120, 620, 720 and 1020 described hereinabove, with the exception that instead of a docking station or ferromagnetic material, tool 1120 comprises indicator assembly 144 for indicating that a minimum quantity of body fluid has been collected into collector 130.

As shown, collector 130 is disposed at application-portion 141 of medium 148, and indicator 146 is disposed at indicator-end 143 of the medium, such that the medium provides fluid communication between the collector and the indicator. In this way, when body fluid 150 is held in collector 130, at least some of the body fluid will pass from the collector, through medium 148, and to indicator 146. For some applications, the body fluid must pass through a length of 10-100 mm of medium 148 in order to reach indicator 146.

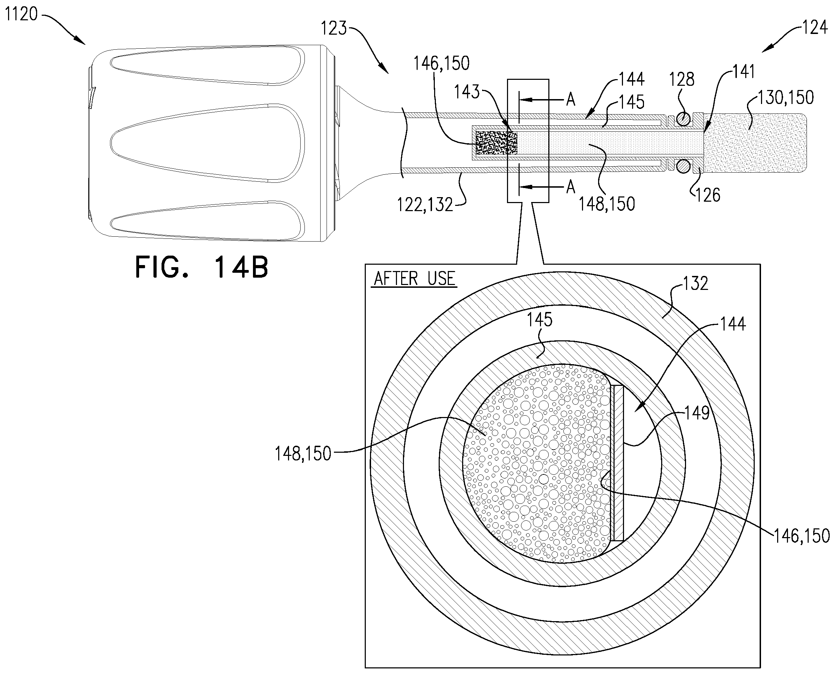

Fig. 14A shows collector 130 and medium 148 in a dry state, such that indicator 146 has a first color. Fig. 14B shows collector 130 and medium 148 in a saturated state, having absorbed body fluid 150, such that body fluid reaches indicator 146, which is shown having a second color.

Typically, the body fluid will pass more slowly through medium 148 than through a similarly sized and shaped portion of collector 130. That is, medium 148 has a greater flow resistance, per cubic millimeter, than that of the collector. For some applications, the flow resistance of medium 148 is such that when collector 130 holds absorbed body fluid 150, less than ten percent (e.g., less than one percent) of the absorbed body fluid will pass from the collector through the medium, toward the indicator.

For some applications, the difference between flow resistance of medium 148 and collector 130 is inherent to the respective properties of the medium and the collector. Alternatively or in addition, the difference between flow resistance of medium 148 and collector 130 is facilitated by the configuration of tool 1120. For some applications, medium 148 expands as it absorbs body fluid (Fig. 13), and as shown in Figs. 14A-B, the medium is housed within a restraining tube 145. For some such applications, restraining tube 145 has an internal width (e.g., between 2 and 10 mm) that is narrower than unconstrained saturated width 140b (Fig. 13) of the medium. In this way, restraining tube 145 prevents the portion of medium 148 from expanding to its unconstrained saturated width 140b (Fig. 14B).

Typically for such applications, an unconstrained saturated volume of the portion of medium 148 that is housed by tube 145 is greater than the internal volume of the tube. For example, the portion of medium 148 may be housed within a part of restraining tube that has an internal volume of 200 - 1000 cubic millimeters.

It is hypothesized by the inventors that preventing medium 148 from expanding to its unconstrained saturated width and/or volume: i) increases the flow resistance of the medium, and ii) directs the body fluid to pass longitudinally toward indicator 146.

It is further hypothesized by the inventors that the rate at which body fluid 150 will pass from collector 130 into medium 148 is dependent upon a degree to which the collector is saturated with the body fluid. Based on experiments conducted by the inventors, only if collector 130 holds a minimum test-quantity of body fluid (e.g., 350 microliters) will the minimum indicator-quantity of body fluid pass from the collector through medium 148 and to indicator 146 within a predetermined test period of 60 seconds or less (such as 30 seconds), resulting in a change in the indicator's color. Conversely, if collector 130 does not hold the minimum quantity of body fluid, the minimum indicator-quantity of body fluid will not reach the indicator, which will therefore not change color during the testing period.

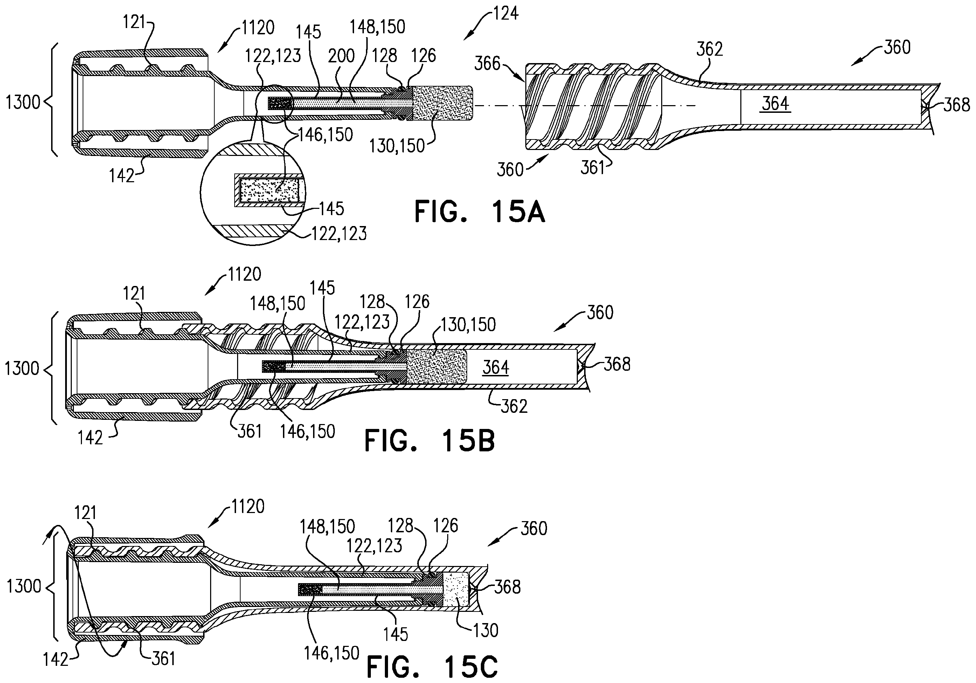

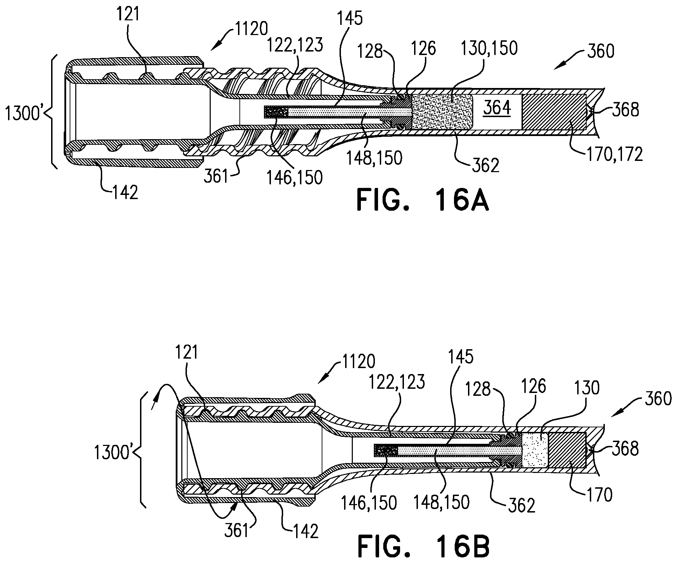

Reference is made to Figs. 15A-C and 16A-B, which are schematic illustrations showing systems 1300, 1300' for use with body fluid 150, in accordance with some applications of the disclosure.

Typically, if indicator 146 indicates that a minute quantity (e.g., 0.5-20 microliters) of body fluid 150 has reached the indicator, a larger quantity of body fluid (e.g., between 100 and 1000 microliters of body fluid) is then extracted from collector 130 to a test apparatus in order to determine a condition of the subject from whom the body fluid was collected.

Figs. 15AC and 16A-B illustrate use of systems 1300, 1300' for extracting body fluid 150 from collector 130, generally in accordance with procedures disclosed in WO 2021/250672 referred to hereinabove with reference to Figs. 9A-C and 10A-B. Fig. 15A shows tool 1120 positioned with distal portion 124 facing a proximal opening 366 of an extractor 360. Typically, and as shown, extractor 360 comprises an elongate barrel 362 that defines a channel 364 therethrough, from opening 366 to a distal outlet 368.

Fig. 15B shows tool 1120 having been advanced distally toward extractor 360, thereby introducing collector 130 into channel 364 via opening 366. As shown in Fig. 15C, further sliding of distal portion 124 through channel 364 compresses collector 130 within the channel. Typically, compressing collector 130 drives body fluid 150 (e.g., some or all of the body fluid) out of the compressed collector and through outlet 368 (e.g., into a test apparatus or a collection tube that is in fluid communication with the outlet).

As shown, body fluid 150 is typically extracted from collector 130 without compressing or otherwise extracting the body fluid from medium 148 or from indicator 146.

Figs 16A-B illustrate use of system 1300' to extract body fluid 150 from collector 130 similarly to as described hereinabove with reference to system 1300, with the exception that a porous carrier 170 is disposed within channel 364. Carrier 170 typically holds a reagent, e.g., a surfactant, and/or a protein such as albumin. In this way, compressing collector 130 within channel 364 drives body fluid 150: (i) out of the collector and though carrier 170, dissolving at least some reagent 172, and (ii) with the dissolved reagent, through outlet 368 and into a test apparatus that is in fluid communication with the outlet.

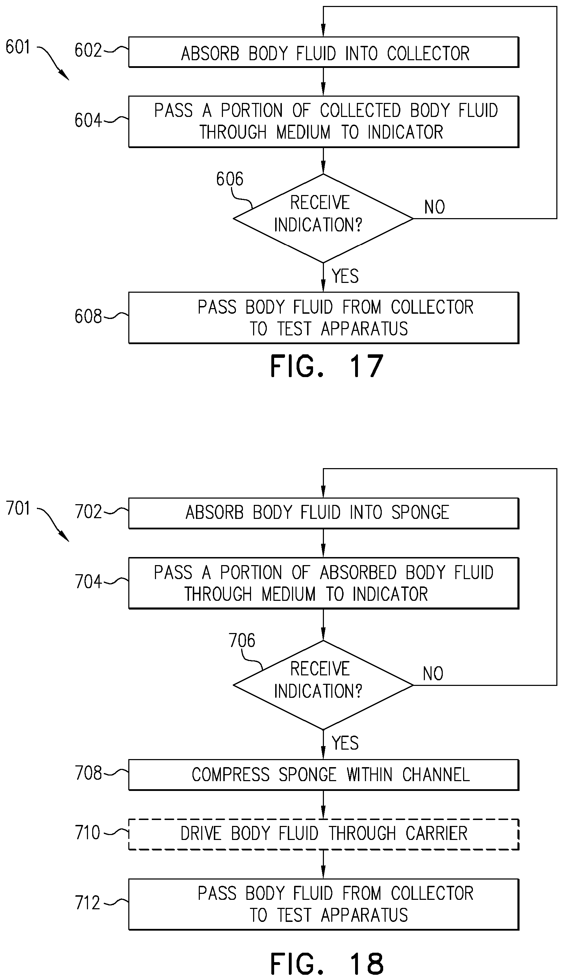

Reference is made to Figs. 17-18, which are flowcharts that schematically illustrate at least some steps of methods 601, 701 that are performed in accordance with some applications of the present disclosure.

As described hereinabove, body fluid 150 is absorbed into collector 130 (steps 602, 702), e.g., from a body of a subject. For some applications, medium 148 is not used to collect the body fluid. A portion of the collected body fluid then typically passes through medium 148 toward indicator 146 (steps 604, 704). The indicator is then observed (steps 606, 706), after the predetermined testing period, in order to determine whether the minimum test-quantity of body fluid 150 has been collected into collector 130. If indicator 146 indicates that body fluid 150 has reached the indicator (e.g., by changing color), it is determined that the minimum test-quantity has been collected into collector 130. If indicator 146 does not yet indicate that body fluid 150 has reached the indicator, it is determined that the minimum test-quantity has not been collected, and additional body fluid is collected (steps 602, 702).

If collector 130 is determined to hold the minimum test-quantity (steps 606, 706), body fluid 150 is typically passed from collector 130 into a test apparatus (steps 608, 712) for testing. For some applications, collector 130 is compressed within a channel 364 (step 708), driving body fluid 150 through an outlet 368 of the channel and into the test apparatus (step 712, Figs. 15B-C, 16A-B). For some such applications, a carrier 170 holding a reagent 172 is disposed within channel 364, such that compressing the collector drives body fluid 150 through the carrier (step 710, Figs. 16A-B), and with dissolved reagent, from outlet 368 and into the test apparatus (step 712). Alternatively or in addition, body fluid 150 may be passed through outlet 368 and into a collection tube for subsequent testing.

## Claims

1. Apparatus for use with a body fluid (150), the apparatus comprising:
a collecting tool (120), the collecting tool:
defining a stem (132) and a pivot-point (133), and
comprising a collector (130) fixedly coupled to the stem (132);
the apparatus **characterized by**
a fulcrum (140) reversibly couplable to the pivot-point (133),
wherein the collecting tool (120) is configured such that while the collecting tool is supported by the fulcrum (140) at the pivot-point (133), a tilt of the collecting tool indicates a quantity of the body fluid (150) collected into the collector (130).

2. The apparatus according to claim 1, wherein:
the collecting tool (120) further comprises an indicator (850) that, while the collecting tool is supported by the fulcrum (140) at the pivot-point (133), displays an indication of the tilt of the collecting tool.

3. The apparatus according to claim 2, wherein:
the indicator (850) is configured to display, while the collecting tool (120) is supported by the fulcrum (140) at the pivot-point (133), a moving indication of the tilt of the collecting tool, such that:
if a predetermined minimum quantity of the body fluid (150) has been collected into the collector (130), a portion of the indicator (850) moves in a first direction, and
if the predetermined minimum quantity of the body fluid (150) has not been collected into the collector (130), the portion of the indicator moves in a second direction.

4. The apparatus according to claim 3, wherein the portion of the indicator (850) is a bubble.

5. The apparatus according to claim 3, wherein the portion of the indicator (850) is configured to slide or roll responsively to the tilt of the collecting tool (120).

6. The apparatus according to any one of claims 1-5, wherein the collector (130) comprises a sponge that is configured to absorb the body fluid (150).

7. The apparatus according to claim 6, further comprising an elongate barrel (362), the barrel shaped to define a channel (364) therethrough, the channel having:
an opening (366) into the channel (364) at a proximal region of the barrel (362), and
an outlet (368) from the channel (364) at a distal region of the barrel (362);
wherein:
the sponge is fixedly coupled to a distal portion of the collecting tool (120); and
the collecting tool (120) is:
configured to introduce the sponge into the channel (364) via the opening (366), and
dimensioned such that sliding the distal portion of the collecting tool (120) distally through the channel (364), while the sponge holds the body fluid (150), compresses the sponge within the channel, driving the body fluid:
out of the compressed sponge and through the outlet (368).

8. The apparatus according to claim 7, further comprising a test apparatus, the test apparatus being in fluid communication with the outlet (368), such that:
sliding the distal portion of the collecting tool (120) distally through the channel (364), while the sponge holds the body fluid (150), compresses the sponge within the channel, driving the body fluid:
out of the compressed sponge, through the outlet (368) and into the test apparatus.

9. The apparatus according to claim 7, further comprising a porous carrier (170) disposed within the channel (368), the carrier holding a reagent, such that:
sliding the distal portion of the collecting tool (120) distally through the channel (364), while the sponge holds the body fluid (150), compresses the sponge within the channel, driving the body fluid:
out of the sponge and though the carrier (170), dissolving at least some of the reagent, and
with the dissolved reagent, through the outlet (368).

10. The apparatus according to claim 9, further comprising a test apparatus, the test apparatus being in fluid communication with the outlet (368), such that:
sliding the distal portion of the collecting tool (120) distally through the channel (364), while the sponge holds the body fluid (150), compresses the sponge within the channel, driving the body fluid:
out of the sponge and though the carrier (170), dissolving at least some of the reagent, and
with the dissolved reagent, through the outlet (368) and into the test apparatus.

11. The apparatus according to any one of claims 1-5, wherein the collecting tool (120) and the fulcrum (140) are configured such that, while the collecting tool is supported by the fulcrum at the pivot-point (133), the collecting tool tilts:
in a first direction, if a predetermined minimum quantity of the body fluid (150) has been collected into the collector (130), and
in a second direction, if the predetermined minimum quantity of the body fluid (150) has not been collected into the collector (130).

12. The apparatus according to claim 11, wherein:
the apparatus is configured to have, while the collecting tool (120) is supported by the fulcrum (140) at the pivot-point (133), a detection sensitivity that represents a minimum difference between:
a quantity of the body fluid (150) in the collector (130) that will cause the collecting tool (120) to tilt in the first direction, and
a quantity of the body fluid (150) in the collector (130) that will cause the collecting tool (120) to tilt in the second direction; and
the detection sensitivity is less than 100 mg.

13. The apparatus according to claim 12, wherein the detection sensitivity is 5-50 mg.

14. The apparatus according to any one of claims 1-5, wherein:
the collecting tool (120) comprises a docking station (222) that defines the pivot-point (133), and
the fulcrum (140) and the docking station (222) are reversibly connectable so as to suspend the collecting tool (120) at the fulcrum.

15. The apparatus according to claim 14, wherein:
the fulcrum (140) and the docking station (222) each comprises a ferromagnetic material, and
the fulcrum (140) and the docking station (222) are reversibly connectable by magnetic attraction between the respective ferromagnetic materials.

## Patentansprüche

1. Einrichtung zur Verwendung mit einem Körperfluid (150), wobei die Einrichtung umfasst:
ein Sammelwerkzeug (120), wobei das Sammelwerkzeug:
einen Schaft (132) und einen Drehpunkt (133) definiert, und
einen Sammler (130) umfasst, der fest an den Schaft (132) gekoppelt ist;
wobei die Einrichtung **gekennzeichnet ist durch** ein Kipplager (140), das reversibel mit dem Drehpunkt (133) koppelbar ist, wobei das Sammelwerkzeug (120) derart konfiguriert ist, dass, während das Sammelwerkzeug durch das Kipplager (140) am Drehpunkt (133) gelagert ist, eine Neigung des Sammelwerkzeugs eine Menge des in den Sammler (130) gesammelten Körperfluids (150) anzeigt.

2. Einrichtung nach Anspruch 1, wobei:
das Sammelwerkzeug (120) ferner eine Anzeige (850) umfasst, die, während das Sammelwerkzeug durch das Kipplager (140) an dem Drehpunkt (133) gelagert ist, eine Angabe der Neigung des Sammelwerkzeugs anzeigt.

3. Einrichtung nach Anspruch 2, wobei:
die Anzeige (850) konfiguriert ist, um, während das Sammelwerkzeug (120) durch das Kipplager (140) am Drehpunkt (133) gelagert ist, eine sich bewegende Anzeige der Neigung des Sammelwerkzeugs anzuzeigen, derart, dass:
wenn eine vorbestimmte Mindestmenge des Körperfluids (150) in den Sammler (130) gesammelt wurde, sich ein Abschnitt der Anzeige (850) in eine erste Richtung bewegt, und
wenn die vorbestimmte Mindestmenge des Körperfluids (150) nicht in den Sammler (130) gesammelt wurde, sich der Abschnitt der Anzeige in eine zweite Richtung bewegt.

4. Einrichtung nach Anspruch 3, wobei der Abschnitt der Anzeige (850) eine Blase ist.

5. Einrichtung nach Anspruch 3, wobei der Abschnitt der Anzeige (850) konfiguriert ist, um sich als Reaktion auf die Neigung des Sammelwerkzeugs (120) zu verschieben oder zu rollen.

6. Einrichtung nach einem der Ansprüche 1-5, wobei der Sammler (130) einen Schwamm umfasst, der konfiguriert ist, das Körperfluid (150) zu absorbieren.

7. Einrichtung nach Anspruch 6, ferner umfassend einen länglichen Zylinder (362), wobei der Zylinder so geformt ist, dass er einen Kanal (364) dorthindurch definiert, wobei der Kanal aufweist:
eine Öffnung (366) in den Kanal (364) an einem proximalen Bereich des Zylinders (362), und
einen Auslass (368) aus dem Kanal (364) an einem distalen Bereich des Zylinders (362);
wobei:
der Schwamm fest an einen distalen Abschnitt des Sammelwerkzeugs (120) gekoppelt ist; und
das Sammelwerkzeug (120):
konfiguriert ist, um den Schwamm über die Öffnung (366) in den Kanal (364) einzuführen, und
so bemessen ist, dass das distale Verschieben des distalen Abschnitts des Sammelwerkzeugs (120) durch den Kanal (364), während der Schwamm das Körperfluid (150) hält, den Schwamm innerhalb des Kanals komprimiert und das Körperfluid:
aus dem komprimierten Schwamm und durch den Auslass (368) treibt.

8. Einrichtung nach Anspruch 7, ferner umfassend eine Testeinrichtung, wobei die Testeinrichtung in Fluidverbindung mit dem Auslass (368) steht, derart, dass:
das distale Verschieben des distalen Abschnitts des Sammelwerkzeugs (120) durch den Kanal (364), während der Schwamm das Körperfluid (150) hält, den Schwamm innerhalb des Kanals komprimiert, wodurch das Körperfluid:
aus dem komprimierten Schwamm durch den Auslass (368) und in die Testeinrichtung getrieben wird.

9. Einrichtung nach Anspruch 7, ferner umfassend einen porösen Träger (170), der in dem Kanal (368) angeordnet ist, wobei der Träger ein Reagenz hält, derart, dass:
das distale Verschieben des distalen Abschnitts des Sammelwerkzeugs (120) durch den Kanal (364), während der Schwamm das Körperfluid (150) hält, den Schwamm innerhalb des Kanals komprimiert, wodurch das Körperfluid:
aus dem Schwamm und durch den Träger (170) getrieben wird, wobei mindestens ein Teil des Reagenzes gelöst wird, und
mit dem gelösten Reagenz durch den Auslass (368) getrieben wird.

10. Einrichtung nach Anspruch 9, ferner umfassend eine Testeinrichtung, wobei die Testeinrichtung in Fluidverbindung mit dem Auslass (368) steht, derart, dass:
das distale Verschieben des distalen Abschnitts des Sammelwerkzeugs (120) durch den Kanal (364), während der Schwamm das Körperfluid (150) hält, den Schwamm innerhalb des Kanals komprimiert, wodurch das Körperfluid:
aus dem Schwamm und durch den Träger (170) getrieben wird, wobei mindestens ein Teil des Reagenzes gelöst wird, und
mit dem gelösten Reagenz durch den Auslass (368) und in die Testeinrichtung getrieben wird.

11. Einrichtung nach einem der Ansprüche 1-5, wobei das Sammelwerkzeug (120) und das Kipplager (140) derart konfiguriert sind, dass, während das Sammelwerkzeug durch das Kipplager an dem Drehpunkt (133) gelagert ist, das Sammelwerkzeug:
in einer ersten Richtung kippt, wenn eine vorbestimmte Mindestmenge des Körperfluids (150) in den Sammler (130) gesammelt wurde, und
in eine zweite Richtung kippt, wenn die vorbestimmte Mindestmenge des Körperfluids (150) nicht in den Sammler (130) gesammelt wurde.

12. Einrichtung nach Anspruch 11, wobei:
die Einrichtung konfiguriert ist, um, während das Sammelwerkzeug (120) durch das Kipplager (140) an dem Drehpunkt (133) gelagert ist, eine Erfassungsempfindlichkeit aufzuweisen, die eine minimale Differenz darstellt zwischen:
einer Menge des Körperfluids (150) in dem Sammler (130), die bewirkt, dass das Sammelwerkzeug (120) in die erste Richtung kippt, und
einer Menge des Körperfluids (150) in dem Sammler (130), die bewirkt, dass das Sammelwerkzeug (120) in die zweite Richtung kippt; und
wobei die Nachweisempfindlichkeit weniger als 100 mg beträgt.

13. Einrichtung nach Anspruch 12, wobei die Nachweisempfindlichkeit 5-50 mg beträgt.

14. Einrichtung nach einem der Ansprüche 1-5, wobei:
das Sammelwerkzeug (120) eine Dockingstation (222) umfasst, die den Drehpunkt (133) definiert, und
das Kipplager (140) und die Dockingstation (222) reversibel verbindbar sind, um das Sammelwerkzeug (120) am Kipplager aufzuhängen.

15. Einrichtung nach Anspruch 14, wobei:
das Kipplager (140) und die Dockingstation (222) jeweils ein ferromagnetisches Material umfassen, und
das Kipplager (140) und die Dockingstation (222) durch magnetische Anziehung zwischen den jeweiligen ferromagnetischen Materialien reversibel verbindbar sind.

## Revendications

1. Appareil pour utilisation avec un fluide corporel (150), l'appareil comprenant :
un outil de collecte (120), l'outil de collecte :
définissant une tige (132) et un point de pivotement (133), et
comprenant un collecteur (130) accouplé fixement à la tige (132) ;
l'appareil **caractérisé par** un point d'appui (140) pouvant être accouplé de manière réversible au point de pivotement (133), dans lequel l'outil de collecte (120) est conçu de telle sorte qu'alors que l'outil de collecte est supporté par le point d'appui (140) au niveau du point de pivotement (133), une inclinaison de l'outil de collecte indique une quantité du fluide corporel (150) collecté dans le collecteur (130).

2. Appareil selon la revendication 1, dans lequel :
l'outil de collecte (120) comprend en outre un indicateur (850) qui, alors que l'outil de collecte est supporté par le point d'appui (140) au niveau du point de pivotement (133), affiche une indication de l'inclinaison de l'outil de collecte.

3. Appareil selon la revendication 2, dans lequel :
l'indicateur (850) est configuré pour afficher, alors que l'outil de collecte (120) est supporté par le point d'appui (140) au niveau du point de pivotement (133), une indication dynamique de l'inclinaison de l'outil de collecte, de telle sorte que :
si une quantité minimale prédéterminée du fluide corporel (150) a été collectée dans le collecteur (130), une partie de l'indicateur (850) se déplace dans une première direction, et
si la quantité minimale prédéterminée du fluide corporel (150) n'a pas été collectée dans le collecteur (130), la partie de l'indicateur se déplace dans une seconde direction.

4. Appareil selon la revendication 3, dans lequel la partie de l'indicateur (850) est une bulle.

5. Appareil selon la revendication 3, dans lequel la partie de l'indicateur (850) est conçue pour coulisser ou rouler en réponse à l'inclinaison de l'outil de collecte (120).

6. Appareil selon l'une quelconque des revendications 1 à 5, dans lequel le collecteur (130) comprend une éponge qui est conçue pour absorber le fluide corporel (150).

7. Appareil selon la revendication 6, comprenant en outre un fourreau (362) allongé, le fourreau étant mis en forme pour définir un canal (364) à travers celui-ci, le canal ayant :
une ouverture (366) dans le canal (364) au niveau d'une région proximale du fourreau (362), et
une sortie (368) du canal (364) au niveau d'une région distale du fourreau (362) ;
dans lequel :
l'éponge est accouplée fixement à une partie distale de l'outil de collecte (120) ; et
l'outil de collecte (120) est :
conçu pour introduire l'éponge dans le canal (364) par l'intermédiaire de l'ouverture (366), et
dimensionné de telle sorte qu'un coulissement de la partie distale de l'outil de collecte (120) distalement à travers le canal (364), alors que l'éponge maintient le fluide corporel (150), comprime l'éponge au sein du canal, en entraînant le fluide corporel :
à l'extérieur de l'éponge comprimée et à travers la sortie (368).

8. Appareil selon la revendication 7, comprenant en outre un appareil de test, l'appareil de test étant en communication fluidique avec la sortie (368), de telle sorte que :
un coulissement de la partie distale de l'outil de collecte (120) distalement à travers le canal (364), alors que l'éponge maintient le fluide corporel (150), comprime l'éponge au sein du canal, en entraînant le fluide corporel :
à l'extérieur de l'éponge comprimée, à travers la sortie (368) et dans l'appareil de test.

9. Appareil selon la revendication 7, comprenant en outre un support (170) poreux disposé au sein du canal (368), le support maintenant un réactif, de telle sorte que :
un coulissement de la partie distale de l'outil de collecte (120) distalement à travers le canal (364), alors que l'éponge maintient le fluide corporel (150), comprime l'éponge au sein du canal, en entraînant le fluide corporel :
à l'extérieur de l'éponge et à travers le support (170), en dissolvant au moins une partie du réactif, et
avec le réactif dissous, à travers la sortie (368).

10. Appareil selon la revendication 9, comprenant en outre un appareil de test, l'appareil de test étant en communication fluidique avec la sortie (368), de telle sorte que :
un coulissement de la partie distale de l'outil de collecte (120) distalement à travers le canal (364), alors que l'éponge maintient le fluide corporel (150), comprime l'éponge au sein du canal, en entraînant le fluide corporel :
à l'extérieur de l'éponge et à travers le support (170), en dissolvant au moins une partie du réactif, et
avec le réactif dissous, à travers la sortie (368) et dans l'appareil de test.

11. Appareil selon l'une quelconque des revendications 1 à 5, dans lequel l'outil de collecte (120) et le point d'appui (140) sont configurés de sorte que, alors que l'outil de collecte est supporté par le point d'appui au niveau du point de pivotement (133), l'outil de collecte s'incline :
dans une première direction, si une quantité minimale prédéterminée du fluide corporel (150) a été collectée dans le collecteur (130), et
dans une seconde direction, si la quantité minimale prédéterminée du fluide corporel (150) n'a pas été collectée dans le collecteur (130).

12. Appareil selon la revendication 11, dans lequel :
l'appareil est configuré pour avoir, alors que l'outil de collecte (120) est supporté par le point d'appui (140) au niveau du point de pivotement (133), une sensibilité de détection qui représente une différence minimale entre :
une quantité du fluide corporel (150) dans le collecteur (130) qui amènera l'outil de collecte (120) à s'incliner dans la première direction, et
une quantité du fluide corporel (150) dans le collecteur (130) qui amènera l'outil de collecte (120) à s'incliner dans la seconde direction ; et
la sensibilité de détection est inférieure à 100 mg.

13. Appareil selon la revendication 12, dans lequel la sensibilité de détection va de 5 à 50 mg.

14. Appareil selon l'une quelconque des revendications 1 à 5, dans lequel :
l'outil de collecte (120) comprend une station d'accueil (222) qui définit le point de pivotement (133), et
le point d'appui (140) et la station d'accueil (222) peuvent être reliés de manière réversible de façon à suspendre l'outil de collecte (120) au niveau du point d'appui.

15. Appareil selon la revendication 14, dans lequel :
le point d'appui (140) et la station d'accueil (222) comprennent chacun un matériau ferromagnétique, et
le point d'appui (140) et la station d'accueil (222) peuvent être reliés de manière réversible par attraction magnétique entre les matériaux ferromagnétiques respectifs.
